(19) Europäisches Patentamt
European Patent Office
Office européen des brevets

(11) Numéro de publication: **0 107 557**
**A1**

(12) # DEMANDE DE BREVET EUROPEEN

(21) Numéro de dépôt: **83401952.3**

(22) Date de dépôt: **06.10.83**

(51) Int. Cl.³: **A 61 K 9/54,** A 61 K 31/40

(30) Priorité: **07.10.82 FR 8216795**

(43) Date de publication de la demande: **02.05.84**
**Bulletin 84/18**

(84) Etats contractants désignés: **AT DE NL**

(71) Demandeur: **LARUELLE, Claude, Avenue Bellevue, F-06270 Villeneuve-Loubet (FR)**

(72) Inventeur: **LARUELLE, Claude, Avenue Bellevue, F-06270 Villeneuve-Loubet (FR)**

(74) Mandataire: **Ores, Irène et al, CABINET ORES 6, Avenue de Messine, F-75008 - Paris (FR)**

(54) **Nouvelle forme galénique du sulpiride, son procédé de préparation et médicament comprenant cette nouvelle forme.**

(57) La présente invention est relative à une nouvelle forme galénique du sulpiride.

Cette nouvelle forme galénique est une forme à libération contrôlée et elle se caractérise en ce qu'elle est constituée par des microgranules comprenant une âme neutre munie d'au moins une couche contenant le sulpiride, puis d'une seconde couche extérieure constituée par une enveloppe microporeuse.

Application: forme galénique retard.

EP 0 107 557 A1

ACTORUM AG

La présente invention est relative à une nouvelle forme galénique du sulpiride, son procédé de préparation et à un médicament comprenant cette nouvelle forme.

Le sulpiride ou le N-[(éthyl-1-pyrrolidinyl-2)méthyl] méthoxy-2-sulfamoyl-5-benzamide de formule développée I

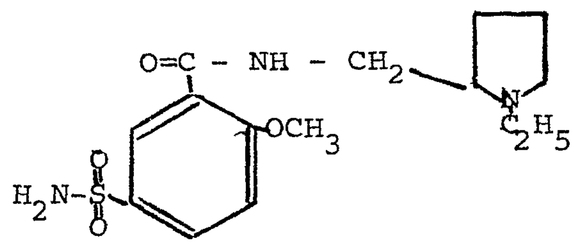

(I)

est connu depuis de nombreuses années et largement utilisé grâce à ses propriétés pharmacologiques remarquables. Ainsi, il trouve actuellement des applications aussi bien pour le traitement des maladies ulcéreuses et gastro-duodénales (dyspepsies, dyskinésies gastro-duodénales et biliaires, gastrites chroniques), en psychopathologie (état dépressif d'étiologie divers, schizophrénie) ainsi qu'en pédiatrie.

Toutefois, l'administration du sulpiride sous ses formes actuelles (solutés injectable ou buvable, gélules, comprimés) présente des inconvénients notables en limitant la commodité d'emploi.

Parmi les inconvénients, il y a lieu de mentionner :

- l'inconvénient de provoquer certaines intolérances, en particulier gastriques, qui empêchent l'utilisation de cet excellent spasmolytique par une catégorie importante de malades d'une part, et d'autre part, qui limitent son utilisation pour les traitements à long terme ;
- l'inconvénient de nécessiter une pluralité de prises quotidiennes qui provoquent une succession d'augmentations et de diminutions rapide des taux plasmatiques, l'organisme étant soumis à des surdosages et à des sous-dosages.

Or, le surdosage peut présenter même un inconvénient grave qui pourrait se manifester par des crises dyskinétiques à type de torticolis spasmodiques, protusion de la langue et le trismus.

La présente invention s'est par conséquent fixé pour but de pourvoir à une nouvelle forme galénique de sulpiride qui répond mieux aux nécessités de la pratique que les formes galéniques antérieurement connues, notamment en ce qu'elle permet de diminuer les doses administrées de sulpiride et rend ainsi possible des administrations prolongées avec une seule prise par jour sans provoquer ni des surdosages, ni des sous-dosages.

La présente invention a pour objet une nouvelle forme galénique du sulpiride à libération contrôlée et programmée caractérisée en ce qu'elle est constituée par des microgranules comprenant une âme neutre constituée d'un grain d'un excipient inerte comprenant au moins deux composants du type appartenant à la classe constituée par le saccharose, l'amidon, le talc, la silice desséchante, le lactose et l'acide stéarique, ce grain neutre étant muni d'au moins une couche comprenant le sulpiride, puis d'une seconde couche extérieure constituée par une enveloppe microporeuse comprenant au moins un polymère naturel et/ou synthétique appartenant à la classe constituée par la gomme laque, la gomme arabique, la gélatine, l'éthyl cellulose, l'acétophtalate de cellulose, le triacétate de cellulose, le polyoxyéthylèneglycol, les méthacrylates, le copolymère styrène-acrylonitrile et la polyvinylpyrrolidone en enveloppes sucessives.

Selon un mode de réalisation avantageux de l'objet de l'invention, l'enveloppe microporeuse est formée de gomme laque (dans une proportion comprise entre 1 et 10 %), l'excipient inerte est constitué d'un mélange saccharose-amidon (dans une proportion de 40-80 % en poids de saccharose et 10 à 40 % en poids d'amidon) et la couche sulpiridique comprend de 1à 20% en poids de sulpiride, de 0,01 à 0,5 % en poids d'acide stéarique, de 5 à 15 % en poids de talc et de 2 à 10 % en poids de silice desséchante.

Selon un autre mode de réalisation avantageux de l'objet de l'invention, l'âme neutre contient également des absorbats de sulpiride.

La présente invention a également pour objet un procédé d'obtention de la nouvelle forme galénique du sulpiride, caractérisé en ce qu'on prépare tout d'abord des microgranules neutres tamisés et séchés, en ce que l'on projette sur cette âme neutre une solution de sulpiride, en ce que l'on enrobe ensuite lesdits microgranules par un enrobage approprié et en ce que l'on forme ensuite l'enveloppe microporeuse.

Selon un mode de réalisation avantageux du procédé objet de la présente invention, le sulpiride est projeté sur l'âme neutre sous forme d'une solution alcoolique.

Selon un autre mode de réalisation avantageux de l'objet de l'invention, le sulpiride forme une pluralité de couches recouvertes chacune par une couche d'enrobage différent ou identique.

La présente invention est en outre relative à des médicaments constitués par ou comprenant la nouvelle forme galénique de sulpiride.

Conformément à l'invention, les médicaments sont constitués par un mélange de microgranules actifs avec les microgranules neutres.

Ce principe de mélanger des microgranules contenant le sulpiride avec les microgranules neutres permet d'obtenir des médicaments à concentration de sulpiride précise et prédéterminée. Les médicaments ainsi obtenus peuvent être présentés sous forme de gélules, de comprimés, de suppositoires, de sirops, de granulés ou de poudre.

Outre les dispositions qui précèdent, l'invention comprend encore d'autres dispositions qui ressortiront de la description qui va suivre qui se réfère à un exemple de préparation du médicament conforme à la présente invention ainsi qu'à un compte-rendu pharmacologique qui met en évidence les nouvelles propriétés de la forme galénique à libération contrôlée conforme à la présente invention.

Il doit être bien entendu, toutefois, que l'exemple de mise en oeuvre qui sera décrit ci-après, de même que le compte-rendu pharmacologique sont donnés uniquement à titre d'illustration de l'objet de l'invention mais

BAD ORIGINAL

ne consitituent en aucune manière une limitation.

1. Exemple de préparation de la nouvelle forme galénique

L'exemple de fabrication correspond à 100.000 gélules dosées à 200 mg de sulpiride.

a) formule de fabrication

| | | |
|---|---|---|
| - sulpiride | 20 | Kg |
| - saccharose | 5,2 | kg |
| - amidon de maïs | 2,8 | kg |
| - acide stéarique | 0,015 | kg |
| - gomme laque | 0,09 | kg |
| - polymères méthacryliques | 0,34 | kg |
| - talc | 0,35 | kg |
| - polyvidone | 0,80 | kg |

- acétone et
- alcool éthylique absolu : qsp pour 100.000 doses de 320 mg environ.

b) procédé de préparation

On granule de l'amidon de maïs et du saccharose puis on tamise et on turbine longuement les grains de façon à les rendre parfaitement sphériques ; on tamise à nouveau et on sèche parfaitement. Dans un mélangeur en acier inoxydable on projette sur les âmes neutres ainsi obtenues une solution alcoolique de sulpiride. On réalise ensuite la première couche en incorporant à ces microgranules les autres excipients à l'exception de la gomme laque, puis on recommence la pulvérisation de sulpiride, cet enrobage étant recommencé plusieurs fois avec tamisage et séchage si nécessaire entre chaque couche.

Lorsque la couche contenant le principe actif est terminée, on réalise la couche extérieure microporeuse en projetant sur les granules la gomme laque en solution dans l'alcool éthylique absolu.

On sèche ensuite soigneusement en éliminant l'alcool éthylique restant, on tamise à nouveau et on contrôle le titre des microgranules obtenus avant de mettre, par exemple, en gélules (après avoir ajusté éventuellement le titrage par addition et homogénéisation avec des microgranules neutres pour arriver au titrage désiré de 200 mg de sulpiride par gélule).

2. Mesure de la libération du sulpiride

L'enveloppe extérieure microporeuse est réalisée de manière à permettre une libération prolongée de sulpiride théorique :

- 1ère heure : libération inférieure ou égale à 60 %
- 4ème heure : libération inférieure ou égale à 90 %
- 8ème heure : libération supérieure à 95 %.

Pour contrôler cette caractéristique, on utilise un appareil à délitement dans lequel on met en contact une quantité de microgranules correspondant à environ 50 mg de principe actif avec des liquides artificiels, l'appareil permettant de maintenir une agitation constante et une température constante de 37° ± 0,5°C. Les liquides artificiels sont des solutions tamponnées à pH successifs utilisés selon le schéma suivant :

| SOLUTIONS | TEMPS DE LIBERATION | pH | RESULTATS Théoriques | RESULTATS Réels |
|---|---|---|---|---|
| 25 ml liquide gastrique | 1 heure (1ère heure) | 1,5 | ≤60 % | 56 % |
| 25 ml liquide intestinal | 1 heure (2ème heure) | 4,5 | >60 % | |
| 25 ml liquide intestinal | 2 h (3 & 4ème heure) | 6,9 | ≤90 % | 80,8% |
| 25 ml liquide intestinal | 2 h (5 & 6ème heure) | 6,9 | >90 % | |
| 25 ml liquide intestinal | 2 h (7 & 8ème heure) | 7,2 | >95 % | 97,3% |

Compte-rendu pharmacologique

La nouvelle forme galénique selon l'invention a fait l'objet d'une étude pharmacocinétique approfondie en comparaison avec la forme comprimé classique. L'étude a été effectuée en essai croisé (cross-over) chez l'homme. Six sujets de sexe masculin recevaient chacune des 2 formes à 2 semaines d'intervalle, une gélule de 200 mg de principe actif contenant les microgranules et 1 comprimé à 200 mg de la forme classique.

La détermination de la concentration plasmatique du sulpiride a été effectuée au moyen de 10 prélèvements dans l'intervalle de 72 heures.

Ces prélèvements veineux ont été de 10 ml et ils ont été effectués aux temps de 0,5 - 1 - 2 - 3 - 4 - 11 - 16 - 24 - 48 - 72 heures après la prise du produit.

Cinq ml ont été centrifugés et le plasma ainsi obtenu a été transféré dans des éprouvettes préalablement silanisées. Ces éprouvettes sont rincées avec une solution de diméthyl-dichlorosilane à 3 % dans le toluène et puis chauffées pendant deux heures à 120°. Le plasma est maintenu dans ces éprouvettes à -20° avant la procédure de quantification.

La quantification du sulpiride a été effectuée par une technique de chromatographie de haute pression et détection électrochimique. Un échantillon de 1 ml additionné au standard interne est injecté dans une colonne chromatographique ( (4 cm x 4 mm Ø) contenant de la résine, l'Amberlite XAD-2.

Cette première procédure permet l'extraction du sulpiride et l'élimination, notamment, des protéines plasmatiques. Le sulpiride retenu sur la colonne est extrait par un gradient de méthanol à pH contrôlé. Le sulpiride et le standard interne sont élués en une fraction de 3 ml d'un mélange à 60 % méthanol/eau.

Après évaporation, les produits sont repris par 90 µl d'eau et 40 µl sont injectés au moyen d'une boucle d'injection sur une colonne de chromatographie de haute pression RP-18 (12 cm x 4 mm Ø int.).

Le solvant d'élution est constitué de 28 % de méthanol et 14 % d'acétonitrile dans un tampon phosphate de 0,05 M à pH 7,98, à un débit de 1 ml/min. pour une pression de 180 $kg/cm^2$ (chromatographe VARIAN LC 4100).

La détection est effectuée par un dispositif électro-chimique avec un potentiel positif de 0.950 volts. Le temps de rétention est de 3 minutes pour le standard interne et de 4,50 minutes pour le sulpiride.

La quantification des produits présents s'effectue par le calcul du rapport de la hauteur du pic du sulpiride et de celle du pic du standard interne.

Au terme de l'étude, les conclusions sont les suivantes :
- la biodisponibilité relative n'est pas modifiée de manière significative ;
- le temps d'apparition du pic sérique passe de 2 heures à 4 heures et demie ;

- la durée de demi-vie passe de 8 heures environ à plus de 12h;
- l'étude des courbes obtenues montre qu'une gélule à 200 mg équivaut à presque deux comprimés de 200 mg.

L'étude toxicologique effectuée sur le rat a permis de déterminer la dose léthale 50 % lorsque les microgranules sont administrés par voie orale :

- chez les femelles, la mortalité est de 40 % à 10 g/kg ;
- chez les mâles, la mortalité est de 40 % à 20 g/kg.

Au plan clinique, la tolérance de la nouvelle présentation a été très bonne et supérieure à la présentation classique. Elle permet ainsi de pratiquer des traitements de longue durée sans inconvénient notable pour le patient.

De plus, du fait de l'économie de 35 à 50 % en dosage de principe actif, on obtient donc une utilisation thérapeutique nettement améliorée.

Il résulte de la description qui précède que quels que soient le mode de mise en oeuvre, de réalisation et d'application adoptés, l'on obtient une nouvelle forme galénique de sulpiride qui présente par rapport aux formes antérieurement connues des avantages importants outre ceux qui ont déjà été mentionnés dans ce qui précède, et notamment :

- elle permet d'assurer une libération du sulpiride de façon contrôlée et indépendamment de la façon dont les microgranules sont administrés avec une courbe de libération du principe actif constante d'un malade à l'autre et d'une prise à l'autre ;
- elle permet une mise en gélules et assure la stabilité du sulpiride, ce qui constitue une amélioration industrielle et médicale importante.

R E V E N D I C A T I O N S

1. Nouvelle forme galénique du sulpiride à libération contrôlée et programmée caractérisée en ce qu'elle est constituée par des microgranules comprenant une âme neutre constituée d'un grain d'un excipient inerte comprenant au moins deux composants du type appartenant à la classe constituée par le saccharose, l'amidon, le talc, la silice desséchante, le lactose et l'acide stéarique, ce grain neutre étant muni d'au moins une couche comprenant le sulpiride, puis d'une seconde couche extérieure constituée par une enveloppe microporeuse comprenant au moins un polymère naturel et/ou synthétique appartenant à la classe constituée par la gomme laque, la gomme arabique, la gélatine, l'éthyl cellulose, l'acétophtalate de cellulose, le triacétate de cellulose, le polyoxyéthylèneglycol, les méthacrylates, le copolymère styrène-acrylonitrile et la polyvinylpyrrolidone en enveloppes successives.

2. Nouvelle forme galénique du sulpiride selon la revendication 1 caractérisée en ce que l'enveloppe microporeuse est formée de gomme laque selon une proportion en poids comprise entre 1 et 10 %.

3. Nouvelle forme galénique du sulpiride selon l'une quelconque des revendications 1 ou 2 caractérisée en ce que l'excipient inerte de l'âme neutre est un mélange comprenant de 40 à 80 % en poids de saccharose et de 10 à 40 % en poids d'amidon.

4. Nouvelle forme selon l'une quelconque des revendications 1 à 3 caractérisée en ce que la couche sulpiridique comprend de 1 à 20 % en poids de sulpiride, de 0,01 à 0,5 % en poids d'acide stéarique, de 5 à 15 % en poids de talc.

5. Nouvelle forme selon l'une quelconque des revendications 1 à 4, caractérisée en ce que l'âme neutre comprend des absorbats de sulpiride.

6. Procédé d'obtention de la nouvelle forme galénique du sulpiride selon l'une quelconque des revendications 1 à 5 caractérisé en ce qu'on prépare tout d'abord des microgranules

neutres tamisés et séchés, en ce que l'on projette sur cette âme neutre une solution de sulpiride, en ce que l'on enrobe ensuite lesdits microgranules par un enrobage approprié et en ce que l'on forme ensuite l'envoloppe microporeuse.

7. Procédé selon la revendication 6 caractérisé en ce que le sulpiride est projeté sur l'âme neutre sous forme d'une solution alcoolique.

8. Procédé selon l'une quelconque des revendications 6 et 7 caractérisé en ce que le sulpiride forme une pluralité de couches recouvertes chacune par une couche d'enrobage différent ou identique.

9. Médicaments constitués par ou comprenant la nouvelle forme galénique de sulpiride, selon l'une quelconque des revendications 1 à 5.

10. Médicaments selon la revendication 9, caractérisés en ce qu'ils sont constitués par un mélange de microgranules actifs avec les microgranules neutres.

0107557

Office européen des brevets

# RAPPORT DE RECHERCHE EUROPEENNE

Numéro de la demande

EP 83 40 1952

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | WO-A-8 201 468 (LARUELLE)<br>* en entier *<br>--- | 1-10 | A 61 K 9/54<br>A 61 K 31/40 |
| Y | THE MERCK INDEX, édition 9, 1976, Merck & Co., no. 8796, RAHWAY N.J. (US)<br>" Sulpiride", page 1165 * en entier *<br>----- | 1-10 | |
| | | | DOMAINES TECHNIQUES RECHERCHES (Int. Cl. 3) |
| | | | A 61 K 9/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 07-12-1983 | BENZ K.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82